(19) Europäisches Patentamt
European Patent Office
Office européen des brevets

(11)  **EP 1 980 620 B1**

(12)  **EUROPÄISCHE PATENTSCHRIFT**

(45) Veröffentlichungstag und Bekanntmachung des Hinweises auf die Patenterteilung:
**06.05.2015  Patentblatt 2015/19**

(51) Int Cl.:
*C12P 13/04* (2006.01)    *C12P 5/02* (2006.01)
*C12P 13/08* (2006.01)

(21) Anmeldenummer: **08102622.1**

(22) Anmeldetag: **14.03.2008**

(54) **Verfahren zur Herstellung von Biogas und Aminosäuren aus einem Hydrolysat**

Process for the poduction of biogas and amino acids from a hydrolysate

Procédé de production de biogaz et d'acides aminés à partir d'un hydrolysat

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **12.04.2007  DE 102007017184**

(43) Veröffentlichungstag der Anmeldung:
**15.10.2008  Patentblatt 2008/42**

(73) Patentinhaber: **Evonik Degussa GmbH 45128 Essen (DE)**

(72) Erfinder:
• **Villela Filho, Murillo 46244, Bottrop-Kirchhellen (DE)**
• **Schaarschmidt, Isabelle 71711, Steinheim (DE)**
• **Wahl, Bernd 76227, Karlsruhe (DE)**
• **Rother, Elmar 64285, Darmstadt (DE)**
• **Zimmermann, Hartmut 88400, Biberach (DE)**
• **Karau, Andreas 63571, Gelnhausen (DE)**

(56) Entgegenhaltungen:
**WO-A2-2005/116228    DE-A1- 10 327 954 US-A1- 2005 153 410**

• **DATABASE WPI Week 198243 Thomson Scientific, London, GB; AN 1982-91807E XP002662364, & JP 57 152890 A (NIPPON KAGAKU KIKAI SEIZO KK) 21. September 1982 (1982-09-21)**

**Beschreibung**

Die Erfindung betrifft ein Verfahren zur integrierten Verwertung der Energie- und Stoffinhalte von bei der enzymatischen Hydrolyse nachwachsender Rohstoffe anfallenden Hydrolysate und Feststoffe, bei dem die entstehende Hydrolyselösung als C-Quelle bei Fermentationen eingesetzt und die nicht hydrolysierten Feststoffe der Biogasgewinnung zugeführt werden.

Stand der Technik

Fermentative Verfahren zur Herstellung von Zielsubstanzen wie z. B. Aminosäuren, Vitaminen und Carotenoiden durch Mikroorganismen sind allgemein bekannt. In Abhängigkeit von den verschiedenen Verfahrensbedingungen werden dabei unterschiedliche Kohlenstoffquellen genutzt. Diese reichen von reiner Saccharose über Rüben- und Zuckerrohmelasse, sogenannten "high test molasses" (invertierte Zuckerrohmelasse) bis hin zu Gluscose aus Stärkehydrolysaten. Für die biotechnologische Herstellung von L-Lysin werden darüber hinaus Essigsäure und Ethanol als großtechnisch einsetzbare Co-Substrate genannt (Pfefferle et al, Biotechnogical Manufacture of Lysine, Advances in Biochemical Engineering/Biotechnology, Vo. 79 (2003), 59-112).

Eine wichtige Kohlenstoffquelle für die Fermentation von Mikroorganismen ist Stärke. Diese muss in vorgelagerten Reaktionsschritten zunächt verflüssigt und verzuckert werden, bevor sie als Kohlenstoffquelle in einer Fermentation genutzt werden kann. Hierzu wird die Särke aus einer natürlichen Stärkequelle wie Kartoffeln, Cassava, Getreide, z. B. Weizen, Mais, Gerste, Roggen oder Reis üblicherweise in vorgereinigter Form gewonnen, anschließend enzymatisch verflüssigt und verzuckert, um dann in der eigentlichen Fermentation zur Produktion der Zielsubstanzen eingesetzt zu werden.

Neuere Techniken befassen sich mit verbesserten Methoden, die die Hertellung von Fermentationsmedien aus erneuerbaren Ressourcen (EP 1205557, US 2002/079268) ermöglichen sollen.

Es wurde auch bereits ein fermentatives Verfahren beschrieben, mit dem es möglich sein soll, Stärke als Kohlenstoffquelle einzusetzen (WO 2005116228).

Bei einer Fermentation entsteht nicht allein das gewünschte Produkt, sondern grundsätzlich auch Biomasse. Diese Biomasse wird als Abfallprodukt entweder entsorgt oder muß sonstwie verwertet werden und verringert durch ihre Entstehung die Ausbeute (Produkt pro Edukt) des Prozesses. Bei der fermentativen Ethanolherstellung werden daher oftmals komplexe Futtermittel als Co-Produkte produziert. Beim so genannten Dry-Milling-Verfahren, durch das ungefähr 65% des Ethanols hergestellt wird, werden allein in den Vereinigten Staaten von Amerika nahezu vier Tonnen an DDGS (Destillers Dried Grains with Solubles) hergestellt (Lyons 2003, Jacques 2003). Zusammenfassend lässt sich der so genannte Dry-Milling-Prozess wie folgt beschreiben: Die Getreidekörner werden in einer Mühle zu feinen Partikeln zermahlen und mit Flüssigkeit gemischt. Dieser Brei wird dann mit einem verflüssigenden Enzym behandelt, um das Getreide zu Dextrinen zu hydrolysieren, die ein Mix aus Oligosacchariden sind. Die Hydrolyse von Stärke mit dem verflüssigenden Enzym, genannt $\alpha$-Amylase, wird oberhalb der Geliertemperatur des Getreides durchgeführt. Der Brei wird bei einer entsprechenden Temperatur gekocht, um die granuläre Struktur der Stärke aufzubrechen und die Gelierung auszulösen. Schließlich werden die entstandenen Dextrine in einem Saccharisierungsprozess mit dem Exoenzym Glucoamylase weiter zu Glucose hydrolysiert. Das dabei anfallende DDGS ist Haupt-Co-Produkt bei der Ethanolherstellung. Circa 80% dieses DDGS wird an Wiederkäuer verfüttert. Dies bedeutet, dass die Verwertung nur wirtschaftlich ist, falls in der Umgebung der Produktionsanlage genug Wiederkäuerzuchtbetriebe als Abnehmer für das DDGS vorhanden sind.

Diese Verwertung von Nebenprodukten der fermentativen Ethanolherstellung als komplexen Futtermitteln muß jedoch von der Herstellung von als Futtermitteladditiven geeigneten Zielsubstanzen unterschieden werden. Bei diesen Verfahren werden zum Beispiel Aminosäuren oder Vitamine als Hauptprodukte fermentativ produziert und finden Anwendung in der Tierernährung. Dabei fallen bei dem Fermentationsprozess zusätzlich komplexe Nebenprodukte an, welche die Biomasse enthalten. Eine Möglichkeit zur Verwertung der Nebenprodukte ist die Herstellung von Düngemitteln aus der Fermentationsbrühe (Ideka 2003). Zur Lysinherstellung werden auch Prozesse eingesetzt, bei denen das Produkt nach der Fermentation nicht aufgereinigt sondern die Biomasse als Bestandteil des Futtermitteladditivs mitverkauft wird (Biolys® US 5,431,933). Als weitere Idee zur Verwertung der anfallenden Biomasse wurde die Biomasserückführung publiziert (Blaesen et. al 2005). Bei diesem Verfahren kann bei Fermentationsprozessen durch Rückführung der anfallenden Biomasse als Edukte in die Fermentation die durchschnittliche Ausbeute erhöht und die zu entsorgende Abfallmenge verringert werden.

Aus dem Stand der Technik ist der anaerobe Abbau von organischer Substanz durch Bakterien bekannt, bei dem Biogas entsteht, das zu 50-85% aus Methan besteht. Die in Biogas gespeicherte und daraus gewonnene Energie wird als erneuerbar bezeichnet, da sie aus nachwachsender organischer Substanz stammt. Zusätzlich ist die energetische Nutzung von Biogas im Gegensatz zur Verbrennung von Erdgas, Erdöl oder Kohle Kohlendioxidneutral, weil sich das entstehende Kohlendioxid im natürlichen Kohlenstoffkreislauf bewegt und von den Pflanzen während ihres Wachstum wieder verbraucht wird. Biogas ist ein hochwertiger Energieträger, das heißt, es kann vielseitig und mit hohem Wirkungsgrad verwertet werden. Die Haupteinnahmequelle bei der Biogasproduktion ist derzeit der Erlös aus der Verstromung. Über die so genannte Kraft-Wärme-Koppelung wird das Biogas klassisch als Kraftstoff im Verbrennungsmotor verwendet, der einen Generator zur Erzeugung von Netzstrom (Wechselstrom beziehungsweise Drehstrom) antreibt. Die gleichzeitig anfallende Motorabwärme aus Kühlung und Abgas kann zum Heizen genutzt werden. Als Alternative zur Stromerzeugung mit höherem Wirkungsgrad werden bereits auch Brennstoffzellen eingesetzt. Biogas kann auch aus Abfällen der L-Lysin-Produktion hergestellt werden (Viesturs et al. 1987 Proc. Latv. Acad. Sci 8 (841):102-105). Ein weiteres Verfahren soll in einem integrierten Prozess die parallele Herstellung von Fleisch (oder Milch), Ethanol, Rinderfutter und Biogas (Biodünger) ermöglichen (US 2005/0153410). Bei der Verwendung von proteinhaltigen Co-Edukten in anaeroben Fermentationsprozessen kann man auf Schwierigkeiten stoßen, da die Proteine bei Änderungen des pH-Wertes in Anwesenheit von divalenten Kationen Strukturänderungen erfahren können, die einen späteren enzymatischen Angriff verhindern können (Mulder 2003, Biological wastewater treatment for industrial effluents; technology and operation, Paques B.V., Balk 3.1 Fermentation).

Der typische Abbauprozess von organischem Material zu Biogas besteht im Wesentlichen aus vier Stufen.

In der ersten Stufe (Hydrolyse) bauen aerobe Bakterien die hochmolekularen organischen Substanzen (Eiweiß, Kohlenhydrate, Fett, Zellulose) mit Hilfe von Enzymen in niedermolekulare Verbindungen wie Einfachzucker, Aminosäuren, Fettsäuren und Wasser um. Die von den hydrolytischen Bakterien ausgeschiedenen Enzyme, haften an der Außenseite der Bakterien (so genannte Exoenzyme) und spalten dabei die organischen Bestandteile des Substrats hydrolytisch in kleine wasserlösliche Moleküle auf. In der zweiten Stufe (Versäuerung) werden die einzelnen Moleküle intrazellulär von säurebildenden Bakterien abgebaut und umgewandelt. Es handelt sich dabei um fakultativ aerobe Spezies, die den noch verbliebenen Sauerstoff verbrauchen und so die für die Methanbakterien nötigen anaeroben Bedingungen schaffen. Hier werden hauptsächlich kurzkettige Fettsäuren, niedermolekulare Alkohole und Gase erzeugt. In der dritten Stufe (Essigsäurebildung) produzieren Essigsäurebakterien aus den organischen Säuren die Ausgangsprodukte für die Methanbildung (Essigsäure, Kohlendioxid und Wasserstoff). In der vierten Stufe (Methanbildung) bilden Methanbakterien das Methan (Eder und Schulz 2006).

Problemstellung

Die Kosten für die Bereitstellung der Kohlenstoffquelle beeinflussen ebenso wie die Energiekosten die Margen im Feinchemikaliengeschäft erheblich. Der größte Posten in den Herstellungskosten von beispielsweise L-Lysin ist die C-Quelle (Pfefferle et al. 2003). Der Zuckerpreis unterliegt starken Schwankungen und hat einen schwerwiegenden Einfluss auf die Wirtschaftlichkeit der Fermentationsprozesse, vor allem bei den niedrigpreisigen Massenprodukten wie Mononatriumglutamat, L-Lysine-HCl und L-Threonin, deren Markt stark vom Wettbewerb bestimmt wird (Ikeda 2003). Die Bereitstellung von für die fermentative Verwertung geeigneten Kohlenstoffquellen aus günstigen Nachwachsenden Rohstoffen ist jedoch technisch nicht trivial. Bei der Aufbereitung dieser Kohlenstoffquellen durch Hydrolyse fallen fasrige Pflanzenreste als zu entsorgende Abfallprodukte an. Zusätzlich entsteht bei der Fermentation Biomasse als zu entsorgendes Abfallprodukt. Wählt man die Biogaserzeugung als Entsorgungsverfahren kann man bei der Verwendung von proteinhaltigen Co-Edukten im dabei eingesetzten anaeroben Fermentationsprozess auf Schwierigkeiten stoßen. Die Proteine können bei Änderungen des pH-Wertes in Anwesenheit von divalenten Kationen Strukturänderungen erfahren die einen späteren enzymatischen Angriff verhindern können (Mulder 2003). Qualitätsschwankungen der Rohstoffe erschweren zusätzlich eine stabile und reproduzierbare Prozessführung bei der Biogasherstellung.

Aufgabenstellung

Es ist Aufgabe der vorliegenden Erfindung, ein integriertes Verfahren zur Herstellung von proteinogenen Aminosäuren aus Nachwachsenden Rohstoffen zur Verfügung zu stellen, bei dem weniger Abfallstoffe anfallen und der Energiegehalt der Rohstoffe besser verwertet wird.

Gegenstand der Erfindung ist ein Verfahren zur integrierten Verwertung der Energie- und Stoffinhalte von bei der enzymatischen Hydrolyse stärkehaltiger nachwachsender Rohstoffe anfallenden Lösungen und Feststoffe, umfassend:

a) die Herstellung einer mindestens eine durch den zur Herstellung einer proteinogenen Aminosäure eingesetzten Mikroorganismus verwertbaren Kohlenstoffquelle enthaltenden wässrigen Hydrolysemischung aus Nachwachsenden Rohstoffen durch enzymatische Hydrolyse,

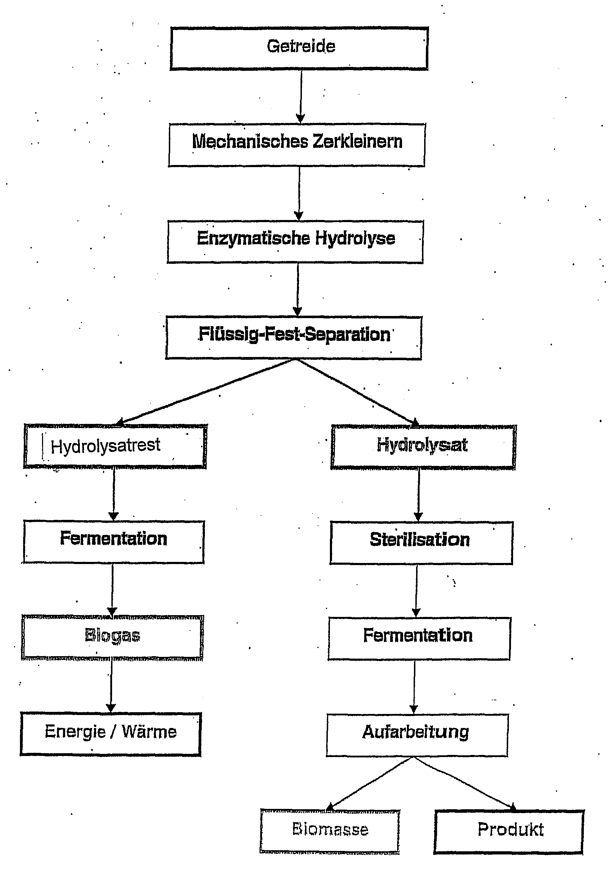

b) die Verwendung dieser wässrigen Mischung bei der Herstellung proteinogener Aminosäuren durch Fermentation, wobei man

c) vor dem Schritt b) die festen Bestandteile aus der in Schritt a) anfallenden Hydrolysemischung abtrennt, und

d) die festen Bestandteile aus der in Schritt a) anfallenden Hydrolysemischung und die in der Fermentation in Schritt b) anfallende Biomasse einer anaeroben Co-Fermentation zur Herstellung von Biogas unterwirft,

wobei man das anfallende Biogas sammelt, verbrennt und in Wärme- und/oder elektrische Energie umwandelt und in einem oder mehreren Verfahrensschritten des Prozesses einsetzt (siehe Abbildung 1).

Als Stärkequelle kommen vor allem trockene Kornfrüchte oder Samen in Betracht, die in getrocknetem Zustand mindestens 40Gew.-% und bevorzugt mindestens 50 Gew.-% Stärkeanteil aufweisen. Diese finden sich in vielen der in großem Maßstab kultivierten Getreidepflanzen wie Mais, Weizen, Hafer, Gerste, Roggen, Reis und verschiedenen Hirsesorten, z. B. Sorghum und Milo. Bevorzugt wird als Stärkequelle Getreide, besonders bevorzugt unter Mais, Roggen und Weizen ausgewählt. Grundsätzlich lässt sich das erfindungsgemäße Verfahren auch mit anderen Stärkequellen durchführen, wie beispielsweise Kartoffeln, Tapioka oder einer Mischung verschiedener stärkehaltiger Früchte oder Samen.

Die durch enzymatische Hydrolyse hergestellten Mischungen enthalten Zucker, bei denen sich vorzugsweise um Monosaccharide wie Hexosen und Pentosen, z. B. Glucose, Fructose, Mannose, Galactose, Sorbose, Xylose, Arabinose und Ribose, insbesondere um Glucose handelt. Der Anteil an von Glucose verschiedenen Monosacchariden kann abhängig von der verwendeten Stärkequelle und den darin enthaltenen nicht-stärkehaltigen Bestandteilen variieren.

Diese Hydrolyseverfahren sind aus dem Stand der Technik bekannt (WO 2005/116228).

Das zuckerhaltige aus der Hydrolysemischung nach Abtrennen der verbleibenden Feststoffe hergestellte Nährmedium wird zur fermentativen Herstellung von proteinogenen Aminosäuren verwendet. Dazu wird die in Schritt a) hergestellte Mischung nach Abtrennung der Feststoffe einer Fermentation gemäß b) zugeführt. Die Durchführung des Fermentationsverfahrens kann in der dem Fachmann bekannten üblichen Art und Weise erfolgen.

Proteinogene Aminosäuren sind bevorzugt Lysin, Methionin, Phenylalanin, Tryptophan und Threonin. Erfindungsgemäß eingesetzte Mikroorganismen werden bevorzugt ausgewählt unter den Gattungen Corynebacterium, Bacillus, Ashbya, Escherichia, Aspergillus, Alcaligenes, Actinobacillus, Anaerobiospirillum, Lactobacillus, Propionibacterium und Clostridium, insbesondere unter Stämmen von Corynebacterium glutamicum, Bacillus subtilis, Ashbya gossypii, Escherichia coli, Aspergillus niger oder Alcaligenes latus, Anaerobiospirillum succiniproducens, Actinobacillus succinogenes, Lactobacillus delbrückii, Lactobacillus leichmanni, Propionibacterium arabinosum, Propionibacterium schermanii, Propionibacterium freudenreichii, Clostridium proionicum und Clostridium acetobutlicum.

In einer bevorzugten Ausführungsform handelt es sich bei der durch Fermentation hergestellten proteinogenen Aminosäure um L-Lysin. Zur Durchführung der Fermentation können hier analoge Bedingungen und Vorgehensweisen angewendet werden, wie sie z. B. bei Pfefferle et al., a. a. O. und in der US 3,708,395 beschrieben werden. Prinzipiell kommen sowohl eine kontinuierliche als auch eine diskontinuierliche (Batch oder Fed-Batch) Betriebsweise in Betracht, bevorzugt ist die Fed-Batch Betriebsweise.

Die Ausführbarkeit des erfindungsgemäßen Verfahrens wird anhand der enzymatischen Hydrolyse von Maisvollkornmehl, feinem Weizenvollkornschrot, Roggenvollkornmehl (Typ 1159) und feinem Roggenvollkornschrot mit drei verschiedenen Enzymen gezeigt. Die Abtrennung der Festphase (Faseranteil) erfolgt durch Filtration mit Druck, Filtration im Zentrifugalfeld, Sedimentation im Zentrifugalfeld sowie Auswaschen. Das aus dem Hydrolysat gewonnene Nährmedium wird im Vakuumrotationsverdampfer aufkonzentriert und anschließend durch Autoklavieren sterilisiert. Die Eignung eines derart prozessierten Weizenhydrolysates als Kohlenstoffquelle für die fermentative Herstellung von Feinchemikalien wurde am Beispiel der L-Lysinherstellung mit dem Stamm Brevibacterium flavum DM1730 (Georgi et al. 2005) sowohl in Schüttelkolbenexperimenten mit absatzweiser Prozessführung als auch im Rührkessel im Zulaufverfahren demonstriert. Dabei zeigte sich überraschenderweise dass die Experimente mit Hydrolysat zu deutlich verbesserten Raum-Zeit-Ausbeuten führten als das Kontrollexperiment mit Standardglucoselösung.

Die Vergärung des abgetrennten Hydrolysatrestes bzw. Fermentationsrückstandes erfolgt nach aus dem Stand der Technik bekannten Verfahren.

Die Beschickung eines Biogasreaktors kann kontinuierlich oder diskontinuierlich erfolgen. Bei der diskontinuierlichen Beschickung, dem so genannten Batch-Prinzip, wird der ganze Faulbehälter auf einmal befüllt. Die Charge fault ohne Substratwechsel bis zum Ende der gewählten Verweilzeit. Dabei setzt die Gasproduktion nach der Füllung ein, erreicht ein Maximum und flacht dann ab. Nach Ablauf der Verweilzeit wird der Behälter bis auf einen als Impfmaterial wirkenden Rest für die nächste Charge vollständig entleert.

Zur zügigen Befüllung und Entleerung ist neben dem Faulbehälter ein Vorrats- und Lagerbehälter in der gleichen Größe notwendig. Die ungleichmäßige Gasproduktion kann durch mehrere kleinere Fermenter, die phasenversetzt befüllt werden, ausgeglichen werden. Mehrere kleinere Behälter verursachen jedoch höhere spezifische Kosten. Nachteilig sind auch die im Vorbehälter während der langen Zeit bis zur Entleerung des Faulbehälters mit Methanverlusten verbundenen Abbauprozesse.

Aus diesen Gründen ist die Nachfrage nach der Batch-Verfahrenstechnologie sehr gering. Sie wird aber für Laborversuche gewählt, um ein Substrat auf sein Verhalten im Fermenter und seine Gasausbeuten zu untersuchen.

Vorteilhaft sind Fermenter, die kontinuierlich mit den zu vergärenden Stoffen beschickt werden, wobei gleichzeitg eine entsprechende Menge von ausgefaultem Substrat abgepumpt wird. Dadurch wird eine permanent stattfindende Faulung mit einer konstanten Gasproduktion erreicht und außerdem einer Versäuerung durch die häufige Zugabe kleiner Substratmengen vorgebeugt.

Die Biogasanlage zur gemeinsamen Vergärung des Hydrolysatrests und des Fermentationsrückstandes wird daher insbesondere als kontinuierlich beschickte Anlage ausgelegt.

Der anaerobe Abbau wird von einer Mischpopulation von Bakterien in verschiedenen Temperaturbereichen vollzogen, wobei man thermophile und mesophile Fahrweisen unterscheidet. Bakterien dieses Typs sind in dem üblichen Faulschlamm aus Kläranlagen enthalten.

Die mesophile Fahrweise erweist sich dabei gegenüber der thermophilen Fahrweise bei Temperaturschwankungen als stabiler.

Eine gute Durchmischung und Verteilung beeinflussen in starkem Maße die Geschwindigkeit und Vollständigkeit des anaeroben Abbaus. Eine kräftige Durchmischung verringert Temperatur- und pH-Wert-Gradienten innerhalb des Fermenters und ermöglicht einen leichten Austritt der Gasbläschen. Gleichzeitig werden Ausbildung von Schwimm- und Sinkschichten verringert oder unterbunden sowie eine gute Durchmischung des Frischsubstrates mit dem Gärsubstrat erreicht. Gegen eine zu starke Durchmischung spricht der hohe Energieaufwand. Außerdem ist für eine gute Methanisierung eine schonende Umwälzung anzustreben, um die Symbiose zwischen den acetogenen und methanogenen Mikroorganismengruppen durch Scherbeanspruchung nicht negativ zu beeinflussen, da sonst die Methanproduktion sinkt.

Die Verweilzeit des Substrates ist eine wichtige Größe zur Dimensionierung des Biogasreaktors. Obwohl die theoretisch notwendige Verweilzeit zum Abbau der Organik in Laborbatchversuchen viel kürzer ist, haben sich in der Praxis 50-80 Tage für die Vergärung von nachwachsenden Rohstoffen und Wirtschaftsdünger bewährt. Ein wichtiger Parameter, der die Verweilzeit einbezieht, ist die sogenannte Faulraumbelastung. Damit wird die Menge an organischer Trockensubstanz bezeichnet, die dem Fermenter täglich zugeführt wird. Sie wird in Kilogramm organische Trockensubstanz pro $m^3$ Fermentervolumen und Tag angegeben. Eine Optimierung der Faulraumbelastung ist einer der wichtigsten Punkte für einen effizienten Betrieb einer Biogasanlage. Eine zu geringe Raumbelastung ist ungünstig, da die gegebenen Kapazitäten nicht voll ausgenutzt werden und eine Biogasanlage somit nicht den maximalen wirtschaftlichen Gewinn abwirft. Eine zu hohe Raumbelastung führt zu einer Instabilität des Fermentationsprozesses, wodurch ein kontinuierlicher Betrieb der Anlage gefährdet ist.

Die Biogasbildung aus dem Weizenhydrolysatrest (Faseranteil), aus dem Fermentationsrückstand (Überschussbiomasse) und aus einer Mischung aus beidem (durch so genannte Co-Fermentation) wurde experimentell überprüft. Im Detail wurde das Biogaspotential, das heißt Zusammensetzung des Biogases und die Menge der Bestandteile, mit einem geeigneten Versuchsaufbau bestimmt. Die Ergebnisse aus den Vergärungen des Weizenhydrolysates und des Fermentationsrückstandes sowie aus der Co-Fermentation wurden verglichen. Dabei zeigte sich, dass bei der Co-Fermentation höhere spezifische Biogaserträge erzielt werden können als bei den gesonderten Fermentationen von Faserresten oder Biomasseabfall allein. Der hohe Proteingehalt der Biomasse hatte dabei überraschenderweise keinen nachteiligen Einfluss auf die Biogaserzeugung.

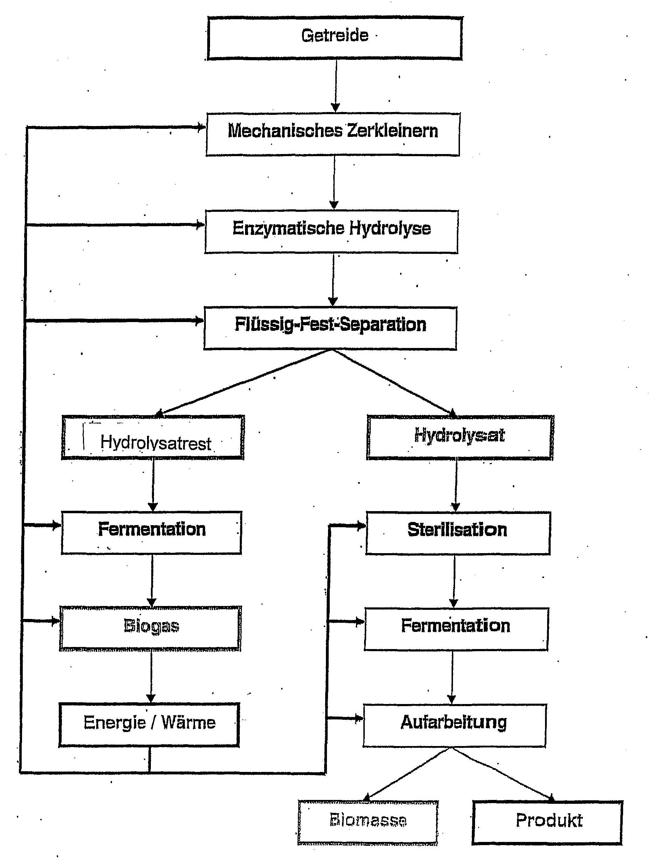

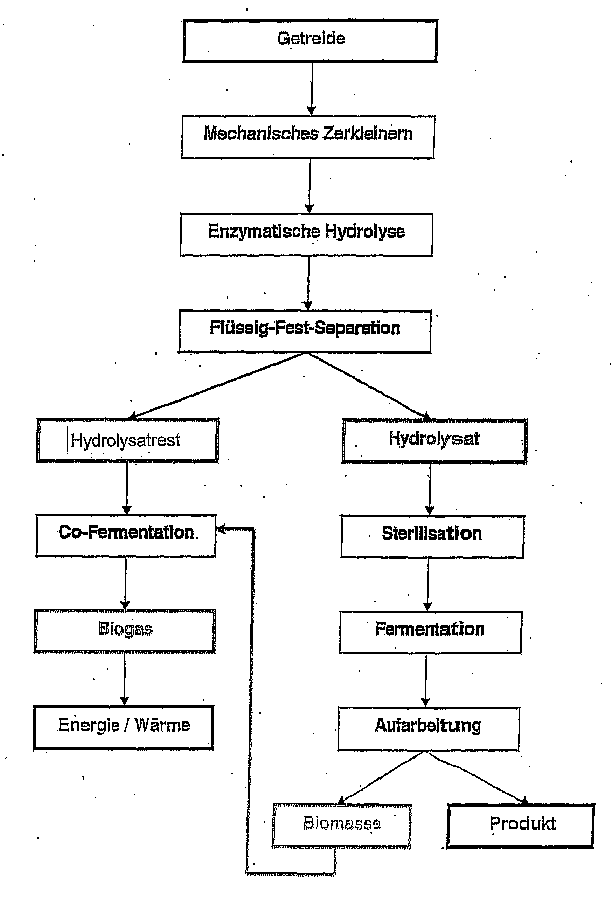

Beim erfindungsgemäßen Verfahren wird die in Schritt d) aus dem Verfahren gemäß Anspruch 1 anfallende Energie / Wärme an einer oder mehreren geeigneten Stellen in den integrierten erfindungsgemäßen Prozess eingeleitet (siehe Abbildung 2). Die bei der aeroben Fermentation zur Herstellung der proteinogenen Aminosäuren anfallende Biomasse wird in die anaerobe Fermentation zur Herstellung des Biogases, dessen verwertbarer Bestandteil aus Methan besteht, eingespeist und dort vergoren (siehe Abbildung 3).

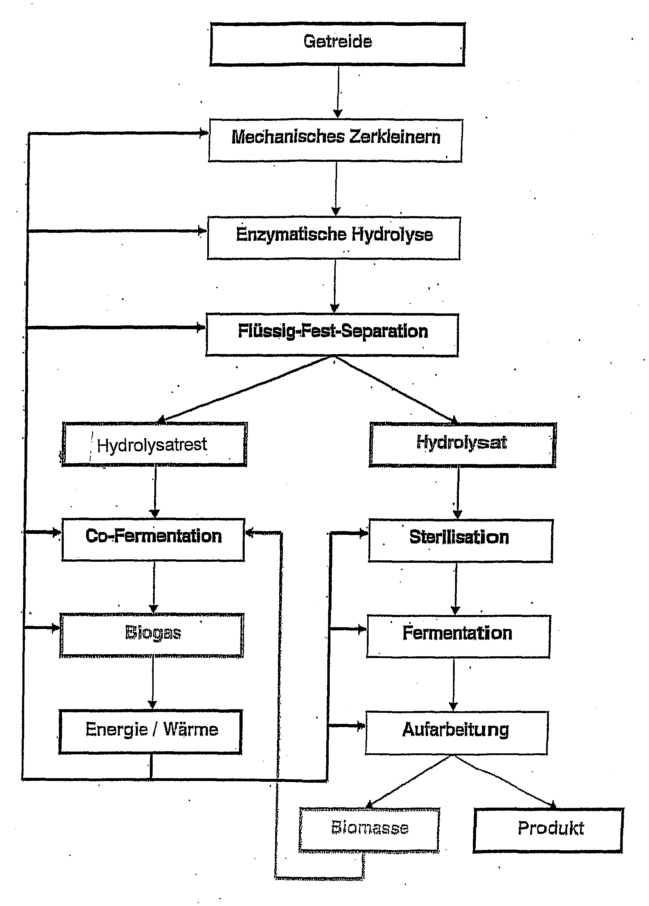

Die vollständigste energetische Ausnutzung und vollständigste Integration bietet der Verfahrensablauf gemäß Abbildung 4.

Dort erfolgt die Einspeisung der im Vergärprozeß anfallenden Energie und Wärme an einer oder mehreren Stellen in den integrierten Prozeß, während gleichzeitig Energie aus der Biomasse in der Vergärstufe gewonnen wird. Der erfindungsgemäße integrierte Prozess erweist sich als kostengünstig da Nachwachsende Rohstoffe eingesetzt werden. Die fasrigen Abfälle, welche bei der Aufbereitung der Nachwachsenden Rohstoffe zu in der Fermentation verwertbaren Kohlenstoffquellen durch enzymatische Hydrolyse anfallen, werden vor der Fermentation abgetrennt, zu Biogas vergoren, das man im allgemeinen weiter in elektrischer Energie oder Wärme umwandelt. Im Speziellen werden mit der gewonnenen Energie oder Wärme zumindest einige Prozessschritte versorgt und somit der Netto-Energiebedarf des integrierten Verfahrens verringert. Die bei der fermentativen Herstellung anfallende Biomasse wird als Co-Edukt in die Biogaserzeugung quer eingespeist und dadurch die Biogasausbeute erhöht. Dabei ist das Co-Edukt für die Biogasherstellung (Biomasse aus der Zielsubstanzherstellung) von vergleichsweise konstanter Qualität, was wiederum eine reproduzierbarere Prozessführung bei der Biogaserzeugung begünstigt. Insgesamt fallen deutlich geringere Mengen an Abfallprodukten und Nebenprodukten an als in bekannten Teilverfahren.

Literatur:

- Blaesen M, Flaschel E, Friehs K (2006) Chem. Ing. Tech 78(3): 267-272

- Busch R, Hirth T, Liese A et al. (2006) Chem. Ing. Tech. 78(3): 219-228

- Eder B, Schulz H (2006) Biogas - Praxis: Grundlagen, Planung, Anlagenbau, Beispiele, Wirtschaftlichkeit, 3rd ed., Ökobuch Verlag, Staufen

- Georgi T, Rittmann D, Wendisch VF (2005) Metab, Eng. 7(4): 291-301

- Ikeda M (2003) Adv. Biochem. Eng. Biotechnol. 79:1-35

- Pfefferle W, Möckel B, Bathe B, Marx A (2003) Adv. Biochem. Eng. Biotechnol. 79:59-112

- Lyons TP (2003) pp. 1-7 in: Jacques KA et al. (eds.) The alcohol textbook, 4th ed. Nottingham University Press, Nottingham

- Mulder R (2003) Biological wastewater treatment for industrial effluents: technology and operation, Paques B. V., Balk

- Viesturs U, Dubrovskis V, Sakse A, Ruklisha M (1987) Proc, Latv. Acad. Sci. 8(481): 102-105

## Versuchsbeschreibung

1.

Tabelle 1: Verwendete Enzyme

| Spirizyme® Plus (Amyloglucosidase) | Novozymes, Dänemark |
|---|---|
| Termamyl® SC (alpha-Amylase) | Novozymes, Dänemark |
| Shearzyme® 500 L (Xylanase) | Novozymes, Dänemark |
| BAN (alpha-Amylase) | Novozymes, Dänemark |

### 2. Enzymatische Hydrolyse

2.1. Es wurden Mehle von Mais (Maisvollkornmehl), von Weizen (Weizenvollkornschrot fein) und von Roggen (Roggenvollkornmehl und Roggenvollkornschrot fein) enzymatisch hydrolysiert. Dazu wurden 300 g Mehl in 610 ml Wasser suspendiert (33% w/w). Als Rührbehälter diente ein Braun B-DCU Bioreaktor, mit pH-Elektrode, Temperaturregelung, Abluftkühler, Scheibenrührer und Stromstörern.

Für die Phasen der Suspendierung, Verflüssigung und Verzuckerung wurden die in Tabelle 1 aufgeführten Enzyme zugegeben und die entsprechenden Parameter eingehalten (siehe Tabelle 2). Zur Einstellung des pH-Wertes wurde 2,5 M Schwefelsäure verwendet. Der Überstand wurde mit dem Messgerät 7100MBS von YSI auf Glucose-Gehalt getestet. Die Reaktion wurde ca. 28 Stunden nach Start des Verflüssigungsschritts beendet.

### Tabelle 2 Parameter der Hydrolyse

| | Zugabe Enzym | pH | T [min] | T [°C] | Rührdrehzahl [rpm] |
|---|---|---|---|---|---|
| Suspendierung | 0,051 mL Shearzyme[1], 0,3 mL BAN[2] | 5,7 | 30 | 55 | 800 1500 (Roggen) |
| Verflüssigung | 0,12 mL Termamyl SC | 5,5-5,8 | 90 | 85 | 1000 |

(fortgesetzt)

|  | Zugabe Enzym | pH | T [min] | T [°C] | Rührdrehzahl [rpm] |
|---|---|---|---|---|---|
| Verzuckerung | 0,270 mL Spirizyme Plus | 4,5-5,0 | ca. 28 | 60 | 1000 |
| [1]: nur bei Weizen und Roggenmehlen [2]: nur bei Roggenmehlen | | | | | |

### 2.2 Auswaschen

Das Auswaschen der Glucose aus dem Feststoffrest wurde batchweise durchgeführt.

Es wurde je Durchgang in VE-Wasser resuspendiert und zentrifugiert. Der Waschvorgang wurde beendet, wenn weniger als ein Prozent der Ausgangskonzentration an Glucose im Waschwasser nachweisbar war.

### 2.3 Aufkonzentrierung

Die Hydrolysate von Weizenvollkornschrot fein, Roggenvollkornmehl und Roggenvollkornschrot fein wurden im Vakuumverdampfer aufkonzentriert. Die Versuche wurden bei unterschiedlichen Temperaturen und angelegten Drücken durchgeführt. Die Massenkonzentration an Glucose in den Lösungen wurde vor und nach dem Aufkonzentrierungsschritt bestimmt.

### 2.4 Sterilisation

Die Sterilisationsversuche wurden mit den aufkonzentrierten Weizenvollkornschrot fein - Glucoselösungen durchgeführt. Es wurden je Versuch 7 g Lösung in eine 100 mL Glasflasche eingewogen. In einem Autklaven mit 75 L Innenraumvolumen, wurden die entsprechenden Haltezeiten und Sterilisationstemperaturen eingestellt. Während des Vorgangs wurden im Autoklaven die Temperatur- und Druck-Daten durch einen Datenlogger aufgenommen. Diese Daten sollten zum Vergleich der verschiedenen Versuche dienen.

Mit einem Spektralphotometer wurde die Extinktion der Lösungen im Bereich der Wellenlängen von 200 nm bis 800nm aufgenommen.

### 3. Massenbilanzierung

Mit der Massenbilanzierung soll die Ausbeute an Glucose pro eingesetzter Rohstoffmenge festgestellt werden. Es soll der Wiederfindungswert dargestellt werden, und die Geschlossenheit der Bilanz gezeigt werden. Dies umschließt die Quantifizierung des Feststoffrestes und, des Anteils an Proteinen, Lipiden, Pentosanen, Glucose und anderen Zuckern in der Lösung

Als Grundlage für die Massenbilanz wurden die Restfeuchte, der Pentosangehalt und der Stärkegehalt der eingesetzten Mehle bestimmt. Die wässrige Phase des Hydrolysats wurde auf Proteingehalt hin untersucht. Es wurde der wässrige Überstand einer Maisvollkornmehl-Hydrolyse analysiert. Die Proteinkonzentration betrug 0,66 mg/mL. Dies entsprach 0,05% der Mehleinwaage. Damit kann davon ausgegangen werden, dass nur vernachlässigbare Mengen des Proteins in Lösung gehen, bzw. dass das nachgewiesene Protein den zugesetzten Enzymen zuzuschreiben ist. Zum Vergleich, der Proteinanteil im Maismehl liegt bei 9,2%. Der Proteinanteil ist somit im Feststoffrest zu vermuten.

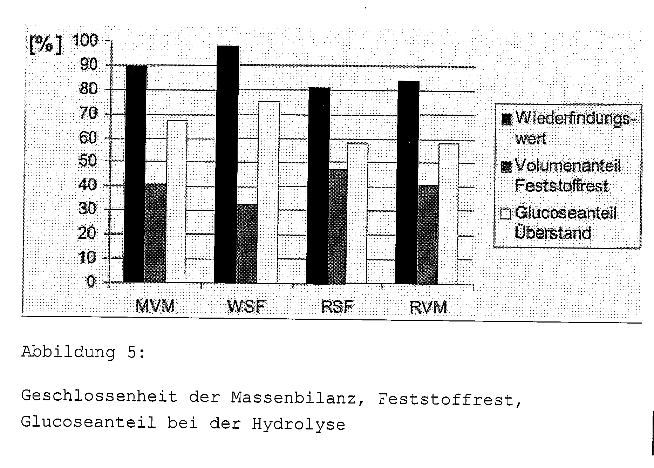

Die Wiederfindungswerte sind in Abbildung 5 dargestellt. Die Geschlossenheit der Massenbilanz war bei Weizenvollkornschrot fein, WSF mit 98% am besten. Die Geschlossenheit lag für MVM bei 90,2% (bei MVM) und für Roggenvollkornmehl und Roggenvollkornschrot fein RMV und RSF bei 83,5% bzw. 81%. In der Abbildung ist weiterhin die Relation des Volumenanteils an Feststoffrest gezeigt. Der Feststoffrest von Weizenvollkornschrot fein war im Verhältnis am geringsten. Dies bedeutet auch, dass der Glucoseanteil im Überstand am größten war. Ebenso ist das Verhältnis von Glucose im Überstand und Feststoffanteil bei den Maisvollkornmehl, Roggenvollkornmehl und Roggenvollkornschrot fein-Hydrolysaten. Je größer das Feststoffvolumen, desto weniger Glucose findet sich im Überstand. Das heißt, dass die Glucoselösung im Feststoff eingeschlossen ist.

### 4. Versuche zur Feststoffabtrennung

Es wurden Zentrifugationsversuche mit den Hydrolyse-Suspensionen von Weizenvollkornschrot fein, Roggenvollkornmehl, Roggenvollkornschrot fein und Maisvollkornmehl durchgeführt. Dabei wurden Proben in graduierten Behältern 20 min bei 4800 RCF zentrifugiert (Minifuge; Heraeus). In weiteren Versuchen wurde das Verhalten bei der Feststoffabtrennung im Weizenvollkornschrot fein-Hydrolysat untersucht (Tabelle 3).

### 4.1 Eigenschaften der Suspension

Tabelle 3

|  | Methode | Gerät |
|---|---|---|
| Viskosität | Rotationsviskosimeter | Haake; Rheostress 600 |
| Leitfähigkeit | Konduktometrisch | WTW; LF 323 |
| Zetapotential | Bestimmung isoelektrischer Punkt | DT 1200 |
| Korngrößenverteilung | Laserbeugungsspektromete | HORIBA LA920 |
| pH-Wert | pH-Elektrode | Mettler-Toledo |
| Chemische Zusammensetzung des Feinanteils | Elementaranalyse |  |
| Pufferwirkung des Hydrolysats | Titration mit 1M Salzsäure | pH-Elektrode; Mettler-Toledo |

### 4.2 Filtration

Filtration der Suspension in einer mit Druck beaufschlagbaren Filterzelle. Die Filterfläche betrug 20 cm$^2$. Der Flux, konnte über eine Waage mit gekoppeltem Erfassungssystem aufgenommen werden (Filtratest®; BOKELA). Nach Ende der Filtration wurden Kuchendicke und TS des Kuchens bestimmt.

### 4.3 Filterzentrifuge

Filtration im Zentrifugalfeld (Megafuge 1.0; Heraeus), in einem Filterbecher, bei einer Schleuderziffer von 1500 g und Zeiten zwischen einer Minute und zehn Minuten. Außerdem wurden Temperatur und pH-Wert variiert (siehe Ergebnisse und Diskussion). Die Filterfläche betrug 15 cm$^2$. Nach Ende des Vorgangs wurden Kuchendicke und TS des Kuchens bestimmt.

### 4.4 Sedimentation

Die Sedimentation wurde im Zentrifugalfeld in graduierten 10 mL-Zentrifugengläsern durchgeführt. Die Schleuderziffer betrug jeweils 3000 g. Variiert wurden pH-Wert, Temperatur und Zentrifugationszeit. Es wurde der Volumenanteil von Überstand und Festphasen dokumentiert, sowie die TS der Festphasen bestimmt.

### 4.5 Biogasbildung aus Hydrolysatrest

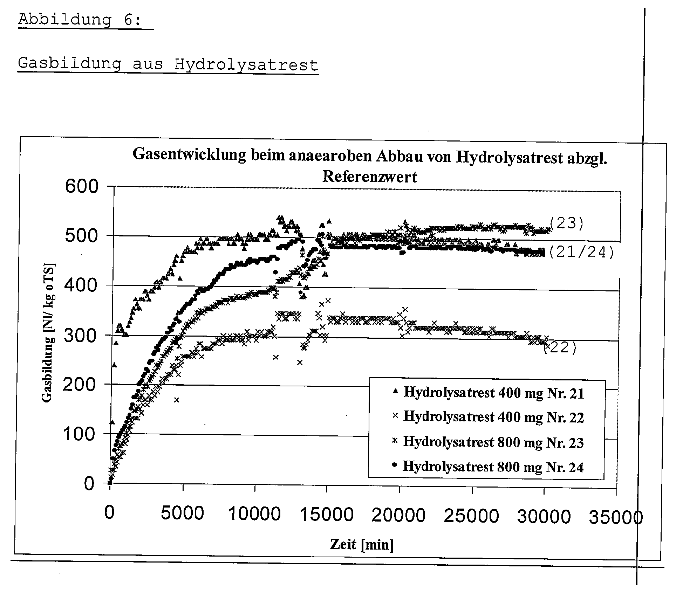

Die Biogasmenge und deren Methangehalt werden mit dem Messsystem OxiTop®-Control im Labormaßstab bestimmt. Die Messung basiert auf dem Druckanstieg durch die Bildung von Biogas in der Messflasche (Abbildung 6).

Zuerst wird das Volumen des Gasraums der eingesetzten Messflaschen inklusive aller Einbauten (Gummiköcher, Magnetrührstab) bestimmt. Der Thermostatenschrank wird auf 35 °C eingestellt und ein Rührbrett eingebaut. Das Zur Verwendung vorgesehene Leitungswasser und der frische Faulschlamm werden einige Minuten mit Stickstoff entgast, um enthaltenen Sauerstoff zu eliminieren, und auf 35 °C temperiert. Vor Versuchsbeginn wird der Faulschlamm zwischen einem und fünf Tagen ausgezehrt.

Die Messflaschen werden mit einem Magnetrührstab auf einem Induktiv-Rührsystem platziert und mit 400 ml des vorbereiteten Leitungswassers befüllt. Danach wird der abgewogene Hydrolysatrest, der insbesondere aus Faseranteilen besteht, zugegeben und durch Begasung der Flaschen mit Stickstoff eine möglichst anaerobe Atmosphäre gewährleistet. Der Faulschlamm enthaltende Behälter wird ebenfalls mit Stickstoff begast. Nach Umrühren werden 50 ml davon mit einer Pipette in die Messflaschen überführt. Nach Messung des pH-Wertes wird dieser gegebenenfalls mit verdünnter Salzsäure oder Natronlauge (0,5 mol/l) auf pH 7 $\pm$ 0,2 korrigiert. Die Flaschen werden verschlossen und nach Aktivierung der Messköpfe bei 35 °C im Dunkeln unter Rühren (ca. 130 U/min) inkubiert. Zur Berücksichtigung der Eigengasbildung des Faulschlammes wird auch ein Versuch ohne Hydrolysatrest gestartet (Versuch 19 in Tabelle 4).

Die Grundlage der Auswertung bildet das ideale Gasgesetz:

$$p \cdot V = n \cdot R \cdot T$$

Damit lässt sich mit dem Druck $p$ [Pa], der Temperatur $T$ [K], der universellen Gaskonstante $R$ [J/(mol·K)] und dem Gasvolumen $V$ [m³] der Flasche die gebildete Menge $n$ [mol] an Gas berechnen.

Die Ergebnisse der gebildeten Gasmengen sind in der Tabelle 4 zusammengefasst:

| Flasche Nr. | 19 | 21 | 22 | 23 | 24 |
|---|---|---|---|---|---|
| Probe | Referenz | 0,4 g Hydrol. | 0,4 g Hydrol. | 0,8 g Hydrol. | 0,8 g Hydrol. |
| Gasvolumen der Flaschen [ml] | 705 | 707 | 708 | 707 | 706 |
| Gasmenge Versuchsbeginn [mol] | 0,0292 | 0,0293 | 0,0293 | 0,0292 | 0,0292 |
| Gesamtgasmenge [mol] | 0,0360 | 0,0375 | 0,0372 | 0,0393 | 0,0394 |
| Menge neu gebildetes Gas [mol] | 0,0068 | 0,0083 | 0,0079 | 0,0101 | 0,0102 |
| Gasmenge aus Hydrolysatrest [mol] | - | 0,0015 | 0,0010 | 0,0033 | 0,0034 |
| Anteil $CH_4$ an Hydrol.gasmenge [%] | - | 41 | 68 | 62 | 58 |
| Anteil $CO_2$ an Hydrol.gasmenge [%] | - | 4 | 15 | 31 | 27 |
| Summe $CO_2$ und $CH_4$ [%] | - | 45 | 84 | 93 | 84 |

Der Anteil an Methan und Kohlendioxid an der aus dem Hydrolysatrest gebildeten Gasmenge liegt bei den Versuchen Nr. 22, Nr. 23 und Nr. 24 zwischen 84 % und 93 %.

**4.6 Biogasbildung aus Co-Vergärung von Hydrolysatrest und Biomasse aus der (Zielprodukt) Fermentation**

Die Versuche werden analog 4.5 durchgeführt. Die insgesamt anfallende Trockensubstanz wird zu 39% aus dem Fermentationsrückstand und zu 61% aus dem Hydrolysatrest gebildet.

Die Tabelle zeigt die gemessenen Methan- und Kohlendioxidanteile in Volumenprozent, sowie die absoluten Mengen abzüglich Eigengasbildung der Referenzprobe. Der Schwefelwasserstoffgehalt wurde nicht bestimmt, da sowohl bei der Vergärung des Hydrolysatrests als auch des Fermentationsrückstands die gaschromatographische Messschwelle von 0,05 Vol.-% unterschritten wurde.

| Tabelle 5 : Methan- und Kohlendioxidmengen der Co-Vergärung | | | |
|---|---|---|---|
| Flasche Nr. | 20 | 21 | 24 |
| Probe | Referenz | 0,4 g Hydrol. & Rückstand | 0,8 g Hydrol. & Rückstand |
| Absolutdruck Versuchsende [hPa] | 1129 | 1194 | 1241 |
| Gasmenge [mol] | 0,0311 | 0,0330 | 0,0342 |
| $CH_4$-Gehalt [Vol.-%] | 1 | 3,7 | 6 |
| $CO_2$-Gehalt [Vol.-%] | 0,9 | 1,8 | 2,5 |
| $CH_4$-Gehalt [mol] | 0,00031 | 0,00122 | 0,00205 |
| $CO_2$-Gehalt [mol] | 0,00028 | 0,00059 | 0,00086 |
| $CH_4$-Menge abzüglich Referenz [mol] | - | 0,00091 | 0,00174 |
| $CO_2$-Menge abzüglich Referenz [mol] | - | 0,00031 | 0,00058 |

**4.7 Vergleich Mono- und Co-Vergärung**

Ein Vergleich der absoluten Methanmengen von 355 Nl /kg oTS (Normliter / kg oTS (eingesetzte organische Trockensubstanz)) bei der anteiligen Addition der Gasmengen der Mono-Vergärungsversuche und 430 Nl /kg oTS beim Co-Vergärungsversuch zeigt, dass durch die Co-Vergärung deutlich höhere massenbezogene Methanmengen erzielt werden kann. Die Co-Vergärung ist damit der Monovergärung zu vorzuziehen.

### 4.8 Abbaugrad des Hydrolysats

Der Abbaugrad gibt an, wie viel Prozent der organischen Trockensubstanz innerhalb der gegebenen Verweilzeit abgebaut wird.

Für diese Berechnung werden die ermittelten Methan-und Kohlendioxidmolmengen in Gewicht umgerechnet und auf die eingesetzte Substratmenge bezogen.

Die Tabelle 6 zeigt den berechneten Abbaugrad des Hydrolysatrestes.

**Tabelle 6: Abbaugrad des Hydrolysatrestes**

| Flasche Nr. | 23 | 24 |
|---|---|---|
| Probe | 0,8 g Hydrolysat | 0,8 g Hydrolysat |
| Menge $CH_4$ [mol] | 0,00205 | 0,00193 |
| Menge $CO_2$ [mol] | 0,00101 | 0,00089 |
| Gewicht $CH_4$ [g] | 0,0328 | 0,0310 |
| Gewicht $CO_2$ [g] | 0,0443 | 0,0394 |
| Gesamtgewicht des Gases [g] | 0,0771 | 0,0704 |
| Menge oTS Hydrolysatrest [g] | 0,139 | 0,139 |
| Abbaugrad [%] | 56 | 51 |

Er zeigt für beide Proben eine gute Übereinstimmung und beträgt im Mittel 54 %. Damit verringert sich die zu entsorgende Menge an Hydrolysatrest (organischen Trockensubstanz) um 54 % durch Koppelung der Biogasproduktion an den Hydrolysenschritt.

### 4.9 Koppelung von Hydrolyse, Fermentation und Biogaserzeugung

Das Beispiel basiert auf einer Hydrolyseanlage mit der Kapazität von 100 000 t/a Glucoseequivalent. Die Anlage ist gekoppelt mit einer Fermentationsanlage zur Herstellung von L-Lysin. Die produzierte Glucoselösung wird auf die für die Fermentation nötige Konzentration eingeengt und sterilisiert werden. Diese Vorgänge sind sehr energieintensiv. Der Energiebedarf einer solche Anlage beträgt ca. 625 GWh/a. Aus der Biogaserzeugung (Co-Vergärung) werden ca. 47,6 GWh/a Strom und ca. 52,3 GWh/a Wärme produziert. Für das vorliegende Beispiel werden somit ca. 1/6 des gesamten Energiebedarfes der Hydrolyse, an die Fermentation zur Herstellung des Zielproduktes gekoppelt ist, durch die Biogaserzeugung nach der erfindungsgemäßen Vorgabe gedeckt.

### Patentansprüche

1. Verfahren zur integrierten Verwertung der Energie- und Stoffinhalte von bei der enzymatischen Hydrolyse stärkehaltiger nachwachsender Rohstoffe anfallender Lösungen und Feststoffe, umfassend:

   a) die Herstellung einer mindestens eine durch den zur Herstellung einer proteinogenen Aminosäure eingesetzten Mikroorganismus verwertbare Kohlenstoffquelle enthaltenden wässrigen Hydrolysemischung aus stärkehaltigen nachwachsenden Rohstoffen durch enzymatische Hydrolyse,
   b) die Verwendung dieser wässrigen Mischung als Kohlenstoffquelle bei Herstellung proteinogener Aminosäuren durch Fermentation, wobei man
   c) vor dem Schritt b) die festen Bestandteile aus der in Schritt a) anfallenden Hydrolysemischung abtrennt, und
   d) die festen Bestandteile aus der in Schritt a) anfallenden Hydrolysemischung und die in der Fermentation in Schritt b) anfallende Biomasse einer anaeroben Co-Fermentation zur Herstellung von Biogas unterwirft, wobei man das anfallende Biogas sammelt, verbrennt und in Wärme- und/oder elektrische Energie umwandelt und in einem oder mehreren Verfahrensschritten des Prozesses einsetzt.

2. Verfahren gemäß Anspruch 1, bei dem man als Kohlenstoffquelle Getreidekörner verwendet.

3. Verfahren gemäß Anspruch 2, bei dem das Getreide ausgewählt ist aus der Gruppe Mais, Roggen, Weizen und Gerste.

**4.** Verfahren nach Anspruch 1, bei dem die Mikroorganismen ausgewählt sind unter den Gattungen Corynebacterium, Bacillus, Ashbya, Escherichia, Aspergillus, Alcaligenes, Actinobacillus, Anaerobiospirillum, Lactobacillus, Propionibacterium und Clostridium.

**5.** Verfahren nach Anspruch 4, bei dem die Mikroorganismen ausgewählt sind unter den Stämmen von Corynebacterium glutamicum, Bacillus subtilis, Ashbya gossypii, Escherichia coli, Aspergillus niger oder Alcaligenes latus, Anaerobiospirillum succiniproducens, Actinobacillus succinogenes, Lactobacillus delbrückii, Lactobacillus leichmanni, Propionibacterium arabinosum, Propionibacterium schermanii, Propionibacterium freudenreichii Clostrididium propionicum und Clostridium acetobutlicum.

**Claims**

**1.** Process for integrated utilization of the energy and material contents of solutions and solids obtained in the enzymatic hydrolysis of starch-containing renewable raw materials, comprising:

a) the preparation of an aqueous hydrolysis mixture comprising at least one carbon source utilizable by the microorganism used to prepare a proteinogenic amino acid from starch-containing renewable raw materials by enzymatic hydrolysis,
b) the use of this aqueous mixture as a carbon source in preparation of proteinogenic amino acids by fermentation, wherein
c) before step b), the solid constituents from the hydrolysis mixture obtained in step a) are removed, and
d) the solid constituents from the hydrolysis mixture obtained in step a) and the biomass obtained in the fermentation in step b) are subjected to an anaerobic cofermentation for the production of biogas, wherein the biogas obtained is collected, combusted and converted to thermal and/or electrical energy and used in one or more process steps of the process.

**2.** Process according to Claim 1, in which the carbon source used is cereal grains.

**3.** Process according to Claim 2, in which the cereal is selected from the group of maize, rye, wheat and barley.

**4.** Process according to Claim 1, in which the microorganisms are selected from the genera Corynebacterium, Bacillus, Ashbya, Escherichia, Aspergillus, Alcaligenes, Actinobacillus, Anaerobiospirillum, Lactobacillus, Propionibacterium and Clostridium.

**5.** Process according to Claim 4, in which the microorganisms are selected from the strains of Corynebacterium glutamicum, Bacillus subtilis, Ashbya gossypii, Escherichia coli, Aspergillus niger or Alcaligenes latus, Anaerobiospirillum succiniproducens, Actinobacillus succinogenes, Lactobacillus delbrueckii, Lactobacillus leichmanni, Propionibacterium arabinosum, Propionibacterium schermanii, Propionibacterium freudenreichii, Clostridium propionicum and Clostridium acetobutlicum.

**Revendications**

**1.** Procédé pour l'utilisation intégrée des teneurs en énergie et en substances de solutions et solides apparaissant dans l'hydrolyse enzymatique de matières premières renouvelables contenant de l'amidon, comprenant :

a) la préparation par hydrolyse enzymatique, à partir de matières premières renouvelables contenant de l'amidon, d'un mélange aqueux d'hydrolyse contenant au moins une source de carbone utilisable par le microorganisme utilisé pour la production d'un acide aminé protéinogène,
b) l'utilisation de ce mélange aqueux comme source de carbone dans la production d'acides aminés protéinogènes par fermentation, dans lequel
c) avant l'étape b) on sépare les composants solides du mélange d'hydrolyse apparaissant dans l'étape a) et
d) on soumet les composants solides provenant du mélange d'hydrolyse apparaissant dans l'étape a) et la biomasse produite dans la fermentation dans l'étape b) à une co-fermentation anaérobie pour la production de biogaz,

en recueillant, brûlant et convertissant en énergie calorique et/ou électrique et en utilisant dans une ou plusieurs étapes du processus le biogaz produit.

2. Procédé selon la revendication 1, dans lequel on utilise comme source de carbone des grains de céréale.

3. Procédé selon la revendication 2, dans lequel la céréale est choisie dans le groupe constitué par le maïs, le seigle, le blé et l'orge.

4. Procédé selon la revendication 1, dans lequel les micro-organismes sont choisis parmi les genres *Corynebacterium, Bacillus, Ashbya, Escherichia, Aspergillus, Alcaligenes, Actinobacillus, Anaerobiospirillum, Lactobacillus, Propionibacterium* et *Clostridium.*

5. Procédé selon la revendication 4, dans lequel les micro-organismes sont choisis parmi les souches de *Corynebacterium glutamicum, Bacillus subtilis, Ashbya gossypii, Escherichia coli, Aspergillus niger* ou *Alcaligenes latus, Anaerobiospirillum succiniproducens, Actinobacillus succinogenes, Lactobacillus delbrückii, Lactobacillus leichmanni, Propionibacterium arabinosum, Propionibacterium schermanii, Propionibacterium freudenreichii, Clostridium propionicum* et *Clostridium acetobutlicum.*

```
                    ┌─────────────────────────┐
                    │        Getreide         │
                    └─────────────────────────┘
                                 │
                                 ▼
                    ┌─────────────────────────┐
      ┌────────────►│ Mechanisches Zerkleinern│
      │             └─────────────────────────┘
      │                          │
      │                          ▼
      │             ┌─────────────────────────┐
      ├────────────►│  Enzymatische Hydrolyse │
      │             └─────────────────────────┘
      │                          │
      │                          ▼
      │             ┌─────────────────────────┐
      ├────────────►│ Flüssig-Fest-Separation │
      │             └─────────────────────────┘
      │                  ╱              ╲
      │                 ▼                ▼
      │        ┌──────────────┐   ┌──────────────┐
      │        │ Hydrolysatrest│   │  Hydrolysat  │
      │        └──────────────┘   └──────────────┘
      │               │                  │
      │               ▼                  ▼
      │        ┌──────────────┐   ┌──────────────┐
      ├───────►│ Fermentation │   │ Sterilisation│◄──┐
      │        └──────────────┘   └──────────────┘   │
      │               │                  │           │
      │               ▼                  ▼           │
      │        ┌──────────────┐   ┌──────────────┐   │
      ├───────►│    Biogas    │   │ Fermentation │◄──┤
      │        └──────────────┘   └──────────────┘   │
      │               │                  │           │
      │               ▼                  ▼           │
      │        ┌──────────────┐   ┌──────────────┐   │
      │        │Energie / Wärme│   │  Aufarbeitung│◄──┤
      │        └──────────────┘   └──────────────┘   │
      │               │                ╱    ╲         │
      └───────────────┴───────────────     ▼    ▼     │
                                 ┌──────────┐ ┌──────────┐
                                 │ Biomasse │ │ Produkt  │
                                 └──────────┘ └──────────┘
```

```
┌─────────────────────────┐
│        Getreide         │
└─────────────────────────┘
             │
             ▼
┌─────────────────────────┐
│ Mechanisches Zerkleinern│
└─────────────────────────┘
             │
             ▼
┌─────────────────────────┐
│  Enzymatische Hydrolyse │
└─────────────────────────┘
             │
             ▼
┌─────────────────────────┐
│  Flüssig-Fest-Separation│
└─────────────────────────┘
        ╱           ╲
       ▼             ▼
┌──────────────┐  ┌──────────────┐
│ Hydrolysatrest│  │  Hydrolysat  │
└──────────────┘  └──────────────┘
       │                 │
       ▼                 ▼
┌──────────────┐  ┌──────────────┐
│Co-Fermentation│◄─│ Sterilisation│
└──────────────┘  └──────────────┘
       │                 │
       ▼                 ▼
┌──────────────┐  ┌──────────────┐
│   Biogas     │  │ Fermentation │
└──────────────┘  └──────────────┘
       │                 │
       ▼                 ▼
┌──────────────┐  ┌──────────────┐
│Energie / Wärme│  │  Aufarbeitung│
└──────────────┘  └──────────────┘
                       ╱      ╲
                      ▼        ▼
               ┌──────────┐ ┌─────────┐
               │ Biomasse │ │ Produkt │
               └──────────┘ └─────────┘
```

```
                    ┌─────────────────────┐
                    │      Getreide       │
                    └─────────────────────┘
                              │
                              ▼
        ┌──────────→┌─────────────────────────┐
        │           │ Mechanisches Zerkleinern │
        │           └─────────────────────────┘
        │                     │
        │                     ▼
        ├──────────→┌─────────────────────────┐
        │           │  Enzymatische Hydrolyse  │
        │           └─────────────────────────┘
        │                     │
        │                     ▼
        ├──────────→┌─────────────────────────┐
        │           │ Flüssig-Fest-Separation  │
        │           └─────────────────────────┘
        │              ╱              ╲
        │             ▼                ▼
        │    ┌──────────────┐    ┌──────────────┐
        │    │ Hydrolysatrest│    │  Hydrolysat  │
        │    └──────────────┘    └──────────────┘
        │             │                 │
        │             ▼                 ▼
        ├──→┌──────────────┐      ┌──────────────┐
        │   │Co-Fermentation│◄─   │ Sterilisation │
        │   └──────────────┘      └──────────────┘
        │             │                 │
        │             ▼                 ▼
        ├──→┌──────────────┐      ┌──────────────┐
        │   │    Biogas     │      │ Fermentation │
        │   └──────────────┘      └──────────────┘
        │             │                 │
        │             ▼                 ▼
        │   ┌──────────────┐      ┌──────────────┐
        └───│ Energie/Wärme │      │ Aufarbeitung │
            └──────────────┘      └──────────────┘
                                    ╱        ╲
                                   ▼          ▼
                           ┌──────────┐  ┌──────────┐
                           │ Biomasse │  │ Produkt  │
                           └──────────┘  └──────────┘
```

Abbildung 5:

Geschlossenheit der Massenbilanz, Feststoffrest,
Glucoseanteil bei der Hydrolyse

Abbildung 6:

Gasbildung aus Hydrolysatrest

**Gasentwicklung beim anaearoben Abbau von Hydrolysatrest abzgl. Referenzwert**

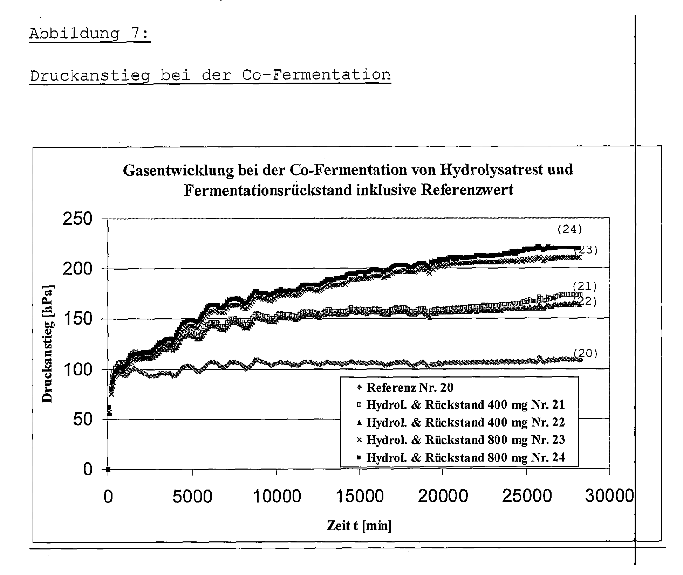

Abbildung 7:

Druckanstieg bei der Co-Fermentation

Gasentwicklung bei der Co-Fermentation von Hydrolysatrest und
Fermentationsrückstand inklusive Referenzwert

19

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1205557 A
- US 2002079268 A
- WO 2005116228 A
- US 5431933 A
- US 20050153410 A
- US 3708395 A, Pfefferle

**In der Beschreibung aufgeführte Nicht-Patentliteratur**

- **PFEFFERLE et al.** Biotechnogical Manufacture of Lysine. *Advances in Biochemical Engineering/Biotechnology,* 2003, vol. 79, 59-112
- **VIESTURS et al.** *Proc. Latv. Acad. Sci,* 1987, vol. 8 (841), 102-105
- **MULDER.** Biological wastewater treatment for industrial effluents; technology and operation. *Paques B.V., Balk 3.1 Fermentation,* 2003
- **BLAESEN M ; FLASCHEL E ; FRIEHS K.** *Chem. Ing. Tech,* 2006, vol. 78 (3), 267-272
- **BUSCH R ; HIRTH T ; LIESE A et al.** *Chem. Ing. Tech.,* 2006, vol. 78 (3), 219-228
- **EDER B ; SCHULZ H.** Biogas - Praxis: Grundlagen, Planung, Anlagenbau, Beispiele, Wirtschaftlichkeit. Ökobuch Verlag, 2006
- **GEORGI T ; RITTMANN D ; WENDISCH VF.** *Metab, Eng.,* 2005, vol. 7 (4), 291-301
- **IKEDA M.** *Adv. Biochem. Eng. Biotechnol.,* 2003, vol. 79, 1-35
- **PFEFFERLE W ; MÖCKEL B ; BATHE B ; MARX A.** *Adv. Biochem. Eng. Biotechnol.,* 2003, vol. 79, 59-112
- **LYONS TP et al.** The alcohol textbook. Nottingham University Press, 2003, 1-7
- **MULDER R.** Biological wastewater treatment for industrial effluents: technology and operation. *Paques B. V., Balk,* 2003
- **VIESTURS U ; DUBROVSKIS V ; SAKSE A ; RUKLISHA M.** *Proc, Latv. Acad. Sci.,* 1987, vol. 8 (481), 102-105